(19) Europäisches Patentamt
European Patent Office
Office européen des brevets

(11) Publication number: **0 150 055**
**B1**

(12) **EUROPEAN PATENT SPECIFICATION**

(45) Date of publication of patent specification: **06.07.88**

(51) Int. Cl.$^4$: **C 07 D 305/08**

(21) Application number: **85100465.5**

(22) Date of filing: **17.01.85**

(54) **Novel substituted trifluorooxetane and preparation thereof.**

(30) Priority: **20.01.84 JP 8768/84**

(43) Date of publication of application:
**31.07.85 Bulletin 85/31**

(45) Publication of the grant of the patent:
**06.07.88 Bulletin 88/27**

(84) Designated Contracting States:
**DE FR GB IT**

(56) References cited:
**WO-A-84/02909**
**DE-A-2 109 966**
**US-A-3 096 344**
**US-A-3 164 610**
**US-A-3 448 121**

(73) Proprietor: **DAIKIN INDUSTRIES, LIMITED**
**No. 1-12-39, Umeda Kita-ku**
**Osaka-shi Osaka-fu (JP)**

(72) Inventor: **Ohsaka, Yohnosuke**
**16-5, Shirakawa 1-chome**
**Ibaraki-shi Osaka-fu (JP)**
Inventor: **Takaki, Shoji**
**11-2-213, Asahigaoka**
**Toyonaka Osaka-fu (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 150 055 B1

## Description

The present invention relates to a novel substituted trifluorooxetane and preparation thereof.

US—A—3 096 344 describes perfluoroalkyloxetanes which may be converted by polymerization or copolymerization to polymeric substances which can be converted by reaction with curing or cross-linking reactants to elastomeric products of high heat stability useful as temperature-resistant elastomers and as flexible temperature-resistant films and coatings.

DE—A—2 109 966 describes homo- or copolymers of 3,3-bis-(heptafluoroisopropoxymethyl)-oxetanes with oil repellant properties.

US—A—3 164 610 describes fluorinated oxetanes having bactericidal properties.

The novel substituted trifluorooxetane of the invention is a cyclic compound of the formula:

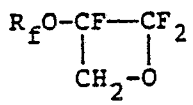 (I)

or

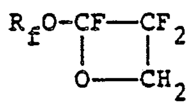 (II)

wherein $R_f$ is $C_1$—$C_{10}$ perfluoroalkyl which optionally contains at least one ether linkage or a group of the formula:

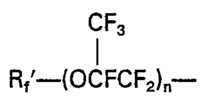 (III)

wherein $R_f'$ is $C_1$—$C_{10}$ perfluoroalkyl which optionally contains at least one ether linkage and n is an integer of 1 to 10.

Perfluoroalkoxytrifluorooxetane (I) or (II) is per se useful as a solvent or a monomer which may be ring opened to produce an oligomer or a polymer. The oligomer or polymer of the substituted trifluorooxetane (I) or (II) finds the same application as conventional fluororesins and perfluoropolyethers.

The substituted trifluorooxetane (I) and (II) may be prepared by reacting a perfluoroether of the formula:

$$R_fOCF{=}CF_2 \quad (IV)$$

wherein $R_f$ is the same as defined above with paraformaldehyde in the presence of hydrogen fluoride.

A molar ratio of the perfluoroether (IV) and paraformaldehyde is usually from 1:0.5 to 1:3, preferably from 1:0.7 to 1:1.5. Hydrogen fluoride is present in the reaction system in an amount of 3 to 20 moles, preferably 5 to 10 moles per mole of paraformaldehyde. The reaction temperature is usually from 50 to 150°C, preferably from 80 to 120°C. Preferably, the reaction is carried out in a liquid phase. The produced substituted trifluorooxetane may be recovered from the reaction mixture by a per se conventional method, for example, by distillation.

The present invention will be hereinafter explained further in detail by the following Examples.

### Example 1

To a 3 liter stainless steel autoclave, perfluoro(propyl vinyl ether) (800 g), paraformaldehyde (180 g) and anhydrous hydrogen fluoride (1,400 g) were charged and reacted with stirring at 80°C for 15 hours.

Thereafter, the reaction mixture was gradually poured into ice-cold water and thoroughly stirred. Then, the organic layer was recovered by liquid-liquid separation, washed with water and treated with soda lime to obtain an organic mixture (680 g), which was distilled under reduced pressure to give a distillate (185 g) containing a compound of the formula:

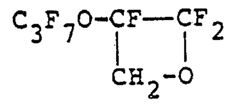 (Ia)

and a compound of the formula:

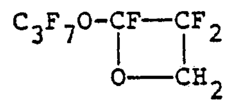 (IIa)

in a molar ratio of about 61:39.

The structures of the compounds were determined by NMR, IR, GC/MS and elemental analysis.

### Example 2

To a 100 ml stainless steel reactor, perfluoro(propyl vinyl ether) (26.6 g), paraformaldehyde (6.0 g) and anhydrous hydrogen fluoride (50 g) were charged and reacted with stirring at 110°C for 12 hours.

Thereafter, the reaction mixture was poured into ice-cold water and stirred. Then, the organic layer was recovered by liquid-liquid separation, washed with water and treated with soda lime to obtain an organic mixture (17.4 g), which was distilled under reduced pressure to give a distillate containing 15.8 mole % of the compound (Ia) and 9.0 mole % of the compound (IIa).

### Example 3

To a 100 ml stainless steel autoclave, perfluoro(propyl vinyl ether) (26.6 g), paraformaldehyde (3.0 g) and anhydrous hydrogen fluoride (30 g) were charged and reacted with stirring at 50°C for 24 hours.

Thereafter, the reaction mixture was treated in the same manner as in Example 1 to obtain a reaction product mixture (22.0 g), which contained 3.0 mole % of the compound (Ia) and 1.7 mole % of the compound (IIa).

### Example 4

To a 100 ml stainless steel autoclave, a compound (30.0 g) of the formula:

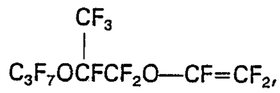

paraformaldehyde (6.0 g) and anhydrous hydrogen fluoride (50 g) were charged and reacted with stirring at 100°C for 5 hours.

Thereafter, the reaction mixture was treated in the same manner as in Example 1 to obtain a reaction product mixture (18.8 g), which was distilled *in vacuo* to give a distillate (3.0 g) containing a compound of the formula:

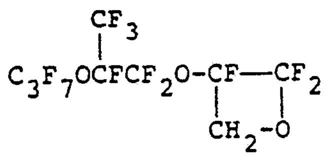

(Ib)

and a compound of the formula:

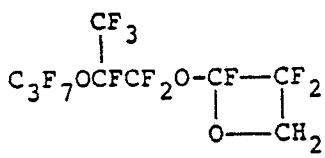

(IIb)

in a molar ratio of about 6:4.

The structures of the compounds were determined by the same methods as in Example 1.

Example 5

To a 100 ml stainless steel autoclave, perfluoro(butyl vinyl ether) (25.0 g), paraformaldehyde (5.0 g) and anhydrous hydrogen fluoride (50 g) were charged and reacted with stirring at 80°C for 20 hours.

Thereafter, the reaction mixture was treated in the same manner as in Example 1 to obtain a reaction product mixture (22.0 g), which was distilled *in vacuo* to give a distillate (4.5 g) containing a compound of the formula:

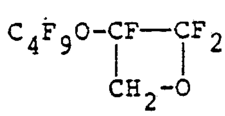

(Ic)

and a compound of the formula:

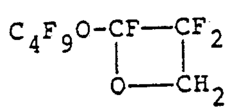

(IIa)

in a molar ratio of about 6:4.

The structures of the compounds were determined by the same methods as in Example 1.

**Claims**

1. A substituted trifluorooxetane of the formula:

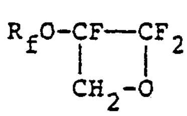

(I)

or

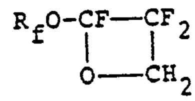

(II)

wherein $R_f$ is $C_1$—$C_{10}$ perfluoroalkyl which optionally contains at least one ether linkage; or a group of the formula:

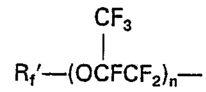

(III)

wherein $R_f'$ is $C_1$—$C_{10}$ perfluoroalkyl which optionally contains at least one ether linkage and n is an integer of 1 to 10.

2. A substituted trifluorooxetane according to claim 1, wherein $R_f$ is $C_1$—$C_{10}$ perfluoroalkyl.

3. A substituted trifluorooxetane according to claim 2, wherein $R_f$ is perfluoropropyl or perfluorobutyl.

4. A substituted trifluorooxetane according to claim 1, wherein $R_f$ is a group of the formula:

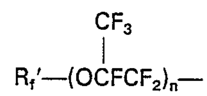

(III)

wherein $R_f'$ and n are the same as defined above.

5. A substituted trifluorooxetane according to claim 4, wherein n is 1.

6. A process for preparing a substituted trifluorooxetane of the formula:

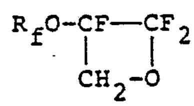

(I)

or

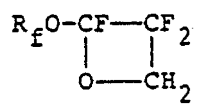

(II)

wherein $R_f$ is $C_1$—$C_{10}$ perfluoroalkyl which optionally contains at least one ether linkage; or a group of the formula:

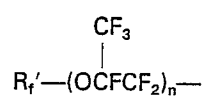

(III)

wherein $R_f'$ is $C_1$—$C_{10}$ perfluoroalkyl which optionally contains at least one ether linkage and n is an integer of 1 to 10, which process comprises reacting a perfluoroether of the formula:

$$R_fOCF{=}CF_2 \qquad (IV)$$

wherein $R_f$ is the same as defined above with paraformaldehyde in the presence of hydrogen fluoride.

7. A process according to claim 6, wherein the molar ratio of the perfluoroether (IV) and paraformaldehyde is from 1:0.5 to 1:3.

8. A process according to claim 7, wherein the molar ratio is from 1:0.7 to 1:1.5.

9. A process according to claim 6, wherein

hydrogen fluoride is used in an amount of 3 to 20 moles per mole of paraformaldehyde.

10. A process according to claim 9, wherein the amount of hydrogen fluoride is 5 to 10 moles per mole of paraformaldehyde.

11. A process according to claim 6, wherein a reaction temperature is from 50 to 150°C.

12. A process according to claim 11, wherein the reaction temperature is from 80 to 120°C.

13. A process according to claim 6, wherein the reaction is carried out in a liquid phase.

**Patentansprüche**

1. Substituiertes Trifluoroxetan der Formel

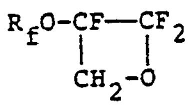 (I)

oder

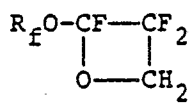 (II)

worin $R_f$ $C_1$—$C_{10}$ Perfluoroalkyl, dans gegebenenfalls wenigstens eine Etherbindung enthält ist, oder eine Gruppe der Formel

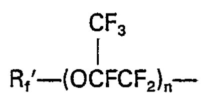 (III)

bedeutet, worin $R_f'$ $C_1$—$C_{10}$ Perfluoralkyl bedeutet, das gewünschtenfalls wenigstens ein Etherbindung enthält und n eine ganze Zahl von 1 bis 10 ist.

2. Substituiertes Trifluoroxetan gemäß Anspruch 1, worin $R_f$ $C_1$—$C_{10}$ Perfluoroalkyl ist.

3. Substituiertes Trifluoroxetan gemäß Anspruch 2, worin $R_f$ Perfluorpropyl oder Perfluorbutyl ist.

4. Substituiertes Trifluoroxetan gemäß Anspruch 1, worin $R_f$ eine Gruppe der Formel

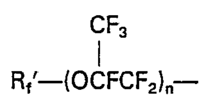 (III)

bedeutet, worin $R_f'$ und n die vorher angegebene Bedeutung haben.

5. Substituiertes Trifluoroxetan gemäß Anspruch 4, worin n 1 ist.

6. Verfahren zur Herstellung eines substituierten Trifluoroxetans der Formel

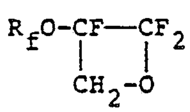 (I)

oder

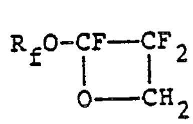 (II)

worin $R_f$ $C_1$—$C_{10}$-Perfluoralkyl bedeutet, das gewünschtenfalls wenigstens eine Etherbindung enthält, oder eine Gruppe der Formel

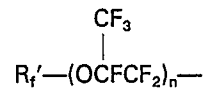 (III)

ist, worin $R_f'$ $C_1$—$C_{10}$-Perfluoralkyl bedeutet, das gewünschtenfalls wenigstens eine Etherbindung enthält und n eine ganze Zahl von 1 bis 10 ist, dadurch gekennzeichnet, daß man ein Perfluorether der Formel

$$R_fOCF{=}CF_2 \quad (IV)$$

worin $R_f$ die vorher angegebene Bedeutung hat, mit Paraformaldehyd in Gegenwart von Fluorwasserstoff umsetzt.

7. Verfahren gemäß Anspruch 6, dadurch gekennzeichnet, daß das Molverhältnis von Perfluorether (IV) und Paraformaldehyd 1:0,5 bis 1:3 ist.

8. Verfahren gemäß Anspruch 7, dadurch gekennzeichnet, daß das Molverhältnis 1:0,7 bis 1:1,5 ist.

9. Verfahren gemäß Anspruch 6, dadurch gekennzeichnet, daß Fluorwasserstoff in einer Menge von 3 bis 20 Mol pro Mol Paraformaldehyd verwendet wird.

10. Verfahren gemäß Anspruch 9, dadurch gekennzeichnet, daß die Menge an Fluorwasserstoff 5 bis 10 Mol pro Mol Paraformaldehyd beträgt.

11. Verfahren gemäß Anspruch 6, dadurch gekennzeichnet, daß die Reaktionstemperatur 50 bis 150°C beträgt.

12. Verfahren gemäß Anspruch 11, dadurch gekennzeichnet, daß die Reaktionstemperatur 80 bis 120°C beträgt.

13. Verfahren gemäß Anspruch 6, dadurch gekennzeichnet, daß die Umsetzung in einer flüssigen Phase durchgeführt wird.

**Revendications**

1. Un trifluorooxéthanne substitué de formule:

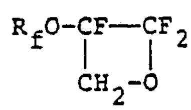 (I)

ou

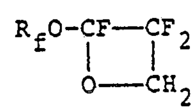 (II)

dans laquelle $R_f$ est un radical perfluoroalkyle en $C_1$—$C_{10}$ qui contient éventuellement au moins une liaison éther; ou un groupe de formule

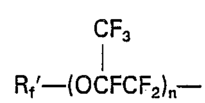 (III)

dans laquelle $R_f'$ est un radical perfluoroalkyle en $C_1$—$C_{10}$ qui contient éventuellement au moins

une liaison éther et n est un nombre entier de 1 à 10.

2. Un trifluorooxéthanne substitué selon la revendication 1, selon laquelle $R_f$ est un radical perfluoroalkyle en $C_1$—$C_{10}$.

3. Un trifluorooxéthanne substitué selon la revendication 2, selon laquelle $R_f$ est le radical perfluoropropyl ou perfluorobutyle.

4. Un trifluorooxéthanne substitué selon la revendication 1, selon laquelle $R_f$ est un groupe de formule:

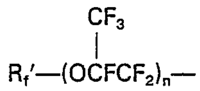 (III)

dans laquelle $R_f'$ et n sont comme définis précédemment.

5. Un trifluorooxéthanne substitué selon la revendication 4, selon laquelle n est égal à 1.

6. Un procédé pour la préparation d'un trifluorooxéthanne substitué de formule:

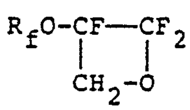 (I)

ou

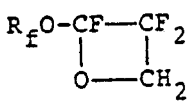 (II)

dans laquelle $R_f$ est un radical perfluoroalkyle en $C_1$—$C_{10}$ qui contient éventuellement au moins une liaison éther; ou un groupe de formule:

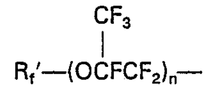 (III)

dans laquelle $R_f'$ est un radical perfluoroalkyle en $C_1$—$C_{10}$ qui contient éventuellement au moins une liaison éther et n est un nombre entier de 1 à 10 ledit procédé comprenant la réaction d'un perfluoroéther de formule:

$$R_fOCF{=}CF_2 \quad \text{(IV)}$$

dans laquelle $R_f$ est le même que celui défini précédemment avec du paraformaldéhyde en présence d'acide fluorhydrique.

7. Un procédé selon la revendication 6, selon laquelle le rapport molaire du perfluoroéther (IV) et du paraformaldéhyde est de 1:0,5 à 1:3.

8. Un procédé selon la revendication 7, selon laquelle le rapport molaire est de 1:0,7 à 1:1,5.

9. Un procédé selon la revendication 6, selon laquelle l'acide fluorhydrique est utilisé en quantité de 3 à 20 moles par mole de paraformaldéhyde.

10. Un procédé selon la revendication 9, selon laquelle la quantité d'acide fluorhydrique est de 5 à 10 moles par mole de paraformaldéhyde.

11. Un procédé selon la revendication 6, selon laquelle le température de réaction est de 50 à 150°C.

12. Un procédé selon la revendication 11, selon laquelle le température de réaction est de 80 à 120°C.

13. Un procédé selon la revendication 6, selon laquelle le réaction est conduite en phase liquide.